# EUROPÄISCHE PATENTSCHRIFT

(11) **EP 2 145 636 B1**
(45) Veröffentlichungstag und Bekanntmachung des Hinweises auf die Patenterteilung: **27.04.2016**
(21) Anmeldenummer: 09012685.5
(22) Anmeldetag: 03.05.2007
(51) Int. Cl.: A61M 1/00

(54) **Absaugpumpeneinheit**
Suction Pump Unit
Unité de pompe d'aspiration

(30) Priorität: 09.05.2006 CH 7492006
(43) Veröffentlichungstag der Anmeldung: 20.01.2010
(62) Teilanmeldung aus: 07720117.6
(73) Patentinhaber: Medela Holding AG, 6340 Baar (CH)
(72) Erfinder: Koch, Urs, 6404 Greppen (CH); RAMELLA, Ivo, 6030 Ebikon (CH)
(74) Vertreter: Clerc, Natalia

(56) Entgegenhaltungen:
- EP-A1- 1 184 043
- GB-A- 2 378 734
- US-A- 3 957 052
- US-A- 4 892 517
- US-A1- 2005 171 495

## Beschreibung

### Technisches Gebiet

Die Erfindung betrifft eine Absaugpumpeneinheit gemäss Oberbegriff des Patentanspruchs 1.

### Stand der Technik

Zum Absaugen von Körperflüssigkeiten oder Sekreten aus Körperkavitäten oder Wunden werden im medizinischen Bereich, insbesondere in der Thoraxdrainage, üblicherweise stationäre Absaugsysteme verwendet. Diese Absaugsysteme bestehen im wesentlichen aus einer Saugquelle, insbesondere einer Vakuumpumpe, einem Fluid- oder Sekretsammelbehälter, einem dazwischen angeordneten Wasserschloss sowie Verbindungsleitungen, namentlich einer vom Patienten zum Sekretbehälter führenden Drainageleitung, einer vom Sekretbehälter zum Wasserschloss führenden Verbindungsleitung und einer das Wasserschloss mit der Saugquelle verbindenden Vakuumleitung.

Diese Absaugsysteme haben sich zwar in der Praxis bewährt. Es ist jedoch für den Heilprozess wesentlich, insbesondere nach einem Eingriff im Thoraxbereich, dass sich der Patient so schnell wie möglich bewegen und sein Bett verlassen kann.

Es wurde deshalb bereits vorgeschlagen, alle oben genannten Bestandteile des Drainagesystems auf einem fahrbaren Gestell zu befestigen, damit der Patient immerhin innerhalb des Spitals eine gewisse Mobilität erlangen kann.

Ferner sind portable Absaugeinheiten bekannt, welche den Mobilitätsbereich des Patienten wesentlich erhöhen. Diese portablen Absaugeinheiten werden mehrheitlich für die Wunddrainage verwendet.

US-A-6'352'525 offenbart jedoch eine portable Pumpeneinheit, welche sich für die Thoraxdrainage eignen soll. Sie lässt sich am Körper des Patienten befestigen und erlaubt ihm somit, sich frei und relativ uneingeschränkt zu bewegen. In dieser Drainagepumpeneinheit ist eine Vakuumpumpe, eine Energiequelle, eine Vakuumkammer und ein Sekretsammelbehälter integriert. Dabei ist die Vakuumkammer in einem ersten Teil, der Sekretsammelbehälter in einem zweiten Teil und die Vakuumpumpe in einem dritten Teil angeordnet. Der erste und dritte Teil sind in Gebrauchslage über dem zweiten Teil angeordnet und lösbar miteinander sowie mit dem zweiten Teil verbunden. Drainageanschlüsse bzw. Vakuumanschlüsse verbinden die entsprechenden Teile miteinander. Diese Vorrichtung ist relativ komplex aufgebaut und lässt sich zudem schlecht reinigen.

WO 99/10024 beschreibt eine portable Pumpeneinheit für Thoraxdrainage, welche über eine externe Leitung mit einem ebenfalls tragbaren nierenförmigen Sekretsammelbehälter verbunden ist. Diese Vorrichtung weist den Nachteil auf, dass zwei getrennte Einheiten getragen werden müssen, welche zudem noch über einen Schlauch miteinander verbunden sind.

Des weiteren offenbart EP-A-1'184'043 eine kleinformatige Absaugpumpe, insbesondere für die Wunddrainage, welche einen Auffangbehälter für abzusaugendes Material und einen Deckel aufweist, wobei alle für den Betrieb erforderlichen Bestandteile der Pumpe im Behälterdeckel integriert sind.

GB 2 378 734 beschreibt eine Absaugpumpe mit einer Pumpe, welche in einem Gehäuse untergebracht ist. An dieses Gehäuse ist ein Kanister anschliessbar, welcher ein Wasserschloss bildet und seinerseits mit einer patientenseitigen Drainageleitung sowie einer zu einem Sekretbehälter führenden Verbindungsleitung verbindbar ist.

Ausserdem zeigt das Dokument US 3,957,052 eine Absaugvorrichtung, bei der mittels einer Spritze Sekret über eine Ventilstruktur in einen Sekretbehälter abgesaugt wird.

### Darstellung der Erfindung

Es ist deshalb eine Aufgabe der Erfindung, eine tragbare Absaugpumpeneinheit zum Absaugen von Körperflüssigkeiten zu schaffen, welche dem Patienten eine grösstmögliche Mobilität gestattet und eine einfache Bedienung der Einheit erlaubt.

Diese Aufgabe löst eine tragbare Absaugpumpeneinheit mit den Merkmalen des Patentanspruchs 1.

Die erfindungsgemässe tragbare Absaugpumpeneinheit zum Absaugen von Körperflüssigkeiten und/oder Luft weist in einer generellen Ausführungsform ein Pumpaggregatgehäuse mit einem Pumpaggregat und mindestens einen lösbar mit diesem Pumpaggregatgehäuse verbundenen Fluidsammelbehälter auf, wobei das Pumpaggregatgehäuse eine vordere Wand, eine Rückwand und eine zwischen diesen zwei Wänden angeordnete Seitenwand aufweist. Ein Adapter für einen Patientenschlauch ist vorhanden, wobei der Adapter in eine Wand des Pumpaggregatgehäuses einführbar ist und wobei der Adapter mit dem Fluidsammelbehälter und vorzugsweise ebenfalls mit dem Pumpaggregat verbindbar ist.

Die erfindungsgemässe tragbare Absaugpumpeneinheit zum Absaugen von Körperflüssigkeiten und/oder Luft weist in einer generellen Ausführungsform ein Pumpaggregatgehäuse mit einem Pumpaggregat und mindestens einen lösbar mit diesem Pumpaggregatgehäuse verbundenen Fluidsammelbehälter auf, wobei das Pumpaggregatgehäuse eine vordere Wand, eine Rückwand und eine zwischen diesen zwei Wänden angeordnete Seitenwand aufweist. In der Seitenwand des Pumpaggregatgehäuses sind ein Vakuumanschluss und ein Sekretanschluss angeordnet.

Die erfindungsgemässe tragbare Absaugpumpeneinheit zum Absaugen von Körperflüssigkeiten und/oder Luft weist in einer generellen Ausführungsform ein Pumpaggregatgehäuse mit einem Pumpaggregat und mindestens einen lösbar mit diesem Pumpaggregatgehäuse verbundenen Fluidsammelbehälter auf, wobei das Pumpaggregatgehäuse eine vordere Wand, eine Rückwand und eine zwischen diesen zwei Wänden angeordnete Seitenwand aufweist. Ein Anschluss für einen Patientenschlauch ist in der vorderen Wand angeordnet.

In einer bevorzugten Ausführungsform weist die tragbare Absaugpumpeneinheit zum Absaugen von Körperflüssigkeiten und/oder Luft ein Pumpaggregatgehäuse mit einem Pumpaggregat und mindestens einen lösbar mit diesem Pumpaggregatgehäuse verbundenen Sekret- oder Fluidsammelbehälter auf. Das Pumpaggregatgehäuse besitzt eine vordere Wand, eine Rückwand und eine zwischen diesen zwei Wänden angeordnete Seitenwand, wobei die vordere Wand und die Rückwand je eine Wandkante aufweisen, welche dieser Seitenwand vorstehen und wobei der Fluidsammelbehälter zwischen diesen Wandkanten gehalten ist. Dadurch ist der Fluidsammelbehälter einfach an das Pumpaggregatgehäuse befestigbar und trotzdem sicher und geschützt darin gehalten.

Der Patient kann die Absaugpumpeneinheit auf verschiedenste Weise mit sich herum tragen. Er kann sie sich an einem Band um den Hals hängen, an einem Gürtel befestigen oder an einem Band über der Schulter tragen. Ist er stationär, so kann er die Einheit auf einen Tisch stellen oder sie sich einfach ans Bett hängen.

Es lassen sich zudem mit demselben Gehäuse verschieden grosse Fluidsammelbehälter verwenden. Dadurch lassen sich die Herstellungs- und die Betriebskosten senken.

In einer bevorzugten Ausführungsform ist der Fluidsammelbehälter zwischen den Wandkanten ein- und ausschwenkbar gehalten. Vorzugsweise ist dabei der Behälter in einem unteren Bereich schwenkbar gehalten und in einem oberen Bereich mit dem Pumpaggregatgehäuse verriegelbar.

Vorzugsweise ist der Behälter vollständig vom Gehäuse entfernbar. Das Auswechseln des Behälters ist erleichtert, wenn der Behälter in das Gehäuse einklinkbar ist.

Die erfindungsgemässe Absaugpumpeneinheit wird für medizinische Zwecke eingesetzt, insbesondere für die Thoraxdrainage und zur Wunddrainage. Andere Anwendungsgebiete sind jedoch möglich, beispielsweise zum Absaugen von Körperflüssigkeiten während eines operativen Eingriffs oder für die Fettabsaugung.

Insbesondere bei der Thorax- und bei der Wunddrainage ist es vorteilhaft, dass das Gerät stets in Betrieb bleiben kann und somit ein Dauervakuum angelegt werden kann, welches aktiv aufrecht erhalten wird. Dies beschleunigt nicht nur die Heilung sondern senkt auch die Betriebskosten, da das Gerät weniger lang benützt und somit weniger lang gemietet werden muss als konventionelle Geräte.

In einer bevorzugten Ausführungsform ist ein Fluidsammelbehälter vorhanden, welcher insbesondere für derartige Absaugpumpeinheiten verwendet werden kann und welcher eine Verschmutzung der Saugleitung bzw. der Saugpumpe durch abgesaugtes Fluid verhindert.

Dieser Fluidsammelbehälter weist einen Sekretanschluss zum Anschluss eines patientenseitigen Drainageschlauchs und einen Vakuumanschluss zum Anschluss an eine Saugpumpe auf. Der Behälter weist einen Innenraum auf, welcher mittels Rippen unterteilt ist, wobei der Innenraum mindestens in eine Vakuumkammer und eine Sekretkammer unterteilt ist, wobei diese zwei Kammern über mindestens einen schmalen Durchgang miteinander verbunden sind und wobei der Vakuumanschluss in der Vakuumkammer und der Sekretanschluss in der Sekretkammer angeordnet sind.

Vorzugsweise sind Vakuumkammer und Sekretkammer nicht direkt miteinander verbunden, sondern es ist eine Zwischenkammer dazwischen geschaltet. Vorzugsweise ist im oberen Bereich der Sekretkammer, unterhalb der Vakuumkammer, noch zusätzlich eine Schrägrippe vorhanden, welche ein Hochschwappen des Fluids verhindert.

Dank der Trennung von Vakuumkammer und Sekretkammer ist der Vakuumanschluss relativ gut geschützt, auch ohne Rückschlagventile oder Membranen.

Weitere vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Es zeigen:
- Figur 1: eine perspektivische Darstellung einer erfindungsgemässen Absaugpumpeneinheit in einer ersten Ausführungsform;
- Figur 2: die Absaugpumpeneinheit gemäss Figur 1 mit einem Einblick in das Innere des Gehäuses;
- Figur 3: die Absaugpumpeneinheit gemäss Figur 1 mit teilweise ausgeschwenktem Fluidsammelbehälter in perspektivischer Ansicht von einer ersten Seite;
- Figur 4: die Absaugpumpeneinheit gemäss Figur 3 in perspektivischer Ansicht von einer zweiten Seite;
- Figur 5: eine Ansicht der Absaugpumpeneinheit gemäss Figur 1 von oben;
- Figur 6: eine Ansicht der erfindungsgemässen Absaugpumpeneinheit in einer zweiten Ausführungsform von oben;
- Figur 7: eine perspektivische Darstellung eines erfindungsgemässen Adapters für einen patientenseitigen Schlauch;
- Figuren 8 und 9: eine perspektivische Darstellung zweier Teile des Gehäuses in einer dritten Ausführungsform;
- Figur 10: eine perspektivische Darstellung eines Fluidsammelbehälters passend zum Gehäuse gemäss Figuren 8 und 9;
- Figur 11: eine perspektivische Darstellung eines ersten Teils des Fluidsammelbehälters gemäss Figur 10 und
- Figur 12: eine perspektivische Darstellung eines zweiten Teils des Fluidsammelbehälters gemäss Figur 10.

### Wege zur Ausführung der Erfindung

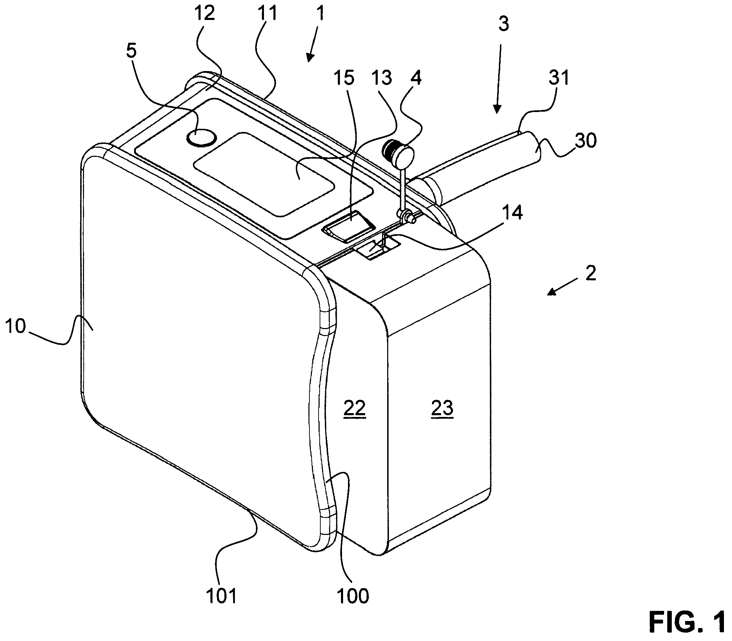

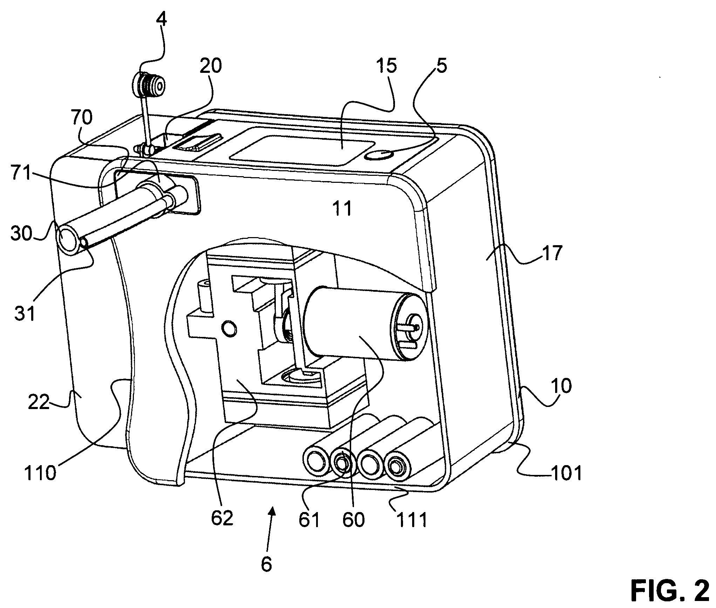

In den Figuren 1 und 2 ist ein erstes Ausführungsbeispiel der erfindungsgemässen Absaugpumpeneinheit dargestellt. Sie besteht im wesentlichen aus einem Pumpaggregatgehäuse 1 mit einem darin angeordneten und in Figur 2 sichtbaren Pumpaggregat 6 und mindestens einem Fluidsammelbehälter 2. Vorzugsweise ist genau ein Fluidsammelbehälter 2 vorhanden. Das Pumpaggregat 6 dient zum Erzeugen des für die Absaugung notwendigen Unterdrucks. Der Fluidsammelbehälter 2 ist mit dem Pumpaggregat 6 verbindbar, so dass im Behälter 2 der Unterdruck erzeugt werden kann. Der Sammelbehälter 2 ist über einen Absaugschlauch bzw. eine Sekretleitung 30 mit einer Kavität oder Wunde des Patienten verbunden, aus welcher die Körperflüssigkeit abgesaugt werden soll, wobei der Behälter 2 die abgesaugte Körperflüssigkeit sammelt.

Vorzugsweise führt nicht nur die Sekretleitung 30 zum Patienten, sondern auch eine Messleitung 31, über welche beispielsweise der Druck oder die Durchflussmenge in der Sekretleitung messbar ist. Vorzugsweise wird hierzu ein doppellumiger Patientenschlauch 3 eingesetzt, welcher beide Leitungen 30, 31 beinhaltet. Der Schlauch 3 kann, wie hier dargestellt, geradlinig aus dem Gehäuse 1 geführt sein. Er kann jedoch auch abgewinkelt angeordnet sein, bzw. es kann ein abgewinkeltes Adapterteil eingesetzt sein, in welches der Schlauch 3 einsteckbar ist.

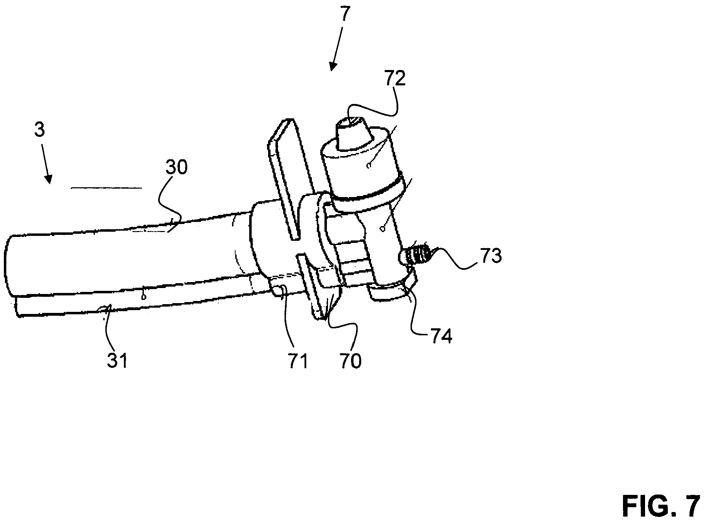

Vorzugsweise wird der Schlauch 3 auf einen Adapter 7 aufgesteckt. Dieser Adapter ist detailliert in Figur 7 dargestellt. Er ist vorzugsweise aus Kunststoff mittels Spritzgusstechnik hergestellt. Er weist ein doppeltes Rohranschlussstück 71 auf, auf oder in welches der doppellumige Patientenschlauch 3 gesteckt werden kann. An dieses Rohranschlussstück 71 ist ein Flansch 70 angeformt, welcher auf dem Gehäuse 1 aufliegt und mittels welcher der Adapter 7 im Gehäuse 1 befestigbar, beispielsweise einklemmbar, ist. Der gehäuseinnenseitige Teil des Adapters 7 weist ein abgewinkeltes Kopplungsteil 72 für einen gehäuseseitigen Sekretanschluss 19 und ein Verbindungsteil 73 für die Messleitung 31 bzw. Messleitungen auf. Das dem Kopplungsteil 72 gegenüberliegende Ende 74 ist geschlossen ausgebildet.

Das Pumpaggregat 6 besteht im wesentlichen aus einem Elektromotor 60, einem Speicherelement 61, hier Batterien, und einer Vakuumpumpe 62. Der Motor 60 ist vorzugsweise an der Pumpe 62 angeflanscht befestigt und die Vakuumpumpe 62 ist vorzugsweise am Gehäuse 1 fixiert gehalten. Als Pumpe eignen sich alle bekannten Pumpen, welche genügend klein ausgebildet sind und die für die entsprechende Anwendung gewünschte Leistung erbringen. Die Durchflussmenge pro Zeit liegt vorzugsweise bei circa 5 l/min. Vorzugsweise wird eine doppelwirkende Membranpumpe verwendet.

Vorzugsweise ist das Aggregat 6 annähernd mittig bzw. zentral im Gehäuse 1 angeordnet. Vorteilhaft ist, wenn durch die Anordnung des Aggregats 6 der gemeinsame Schwerpunkt des Gehäuses 1 und des Aggregats 6 so gewählt ist, dass das Gehäuse 1 beim Tragen nicht auf eine Seite kippt.

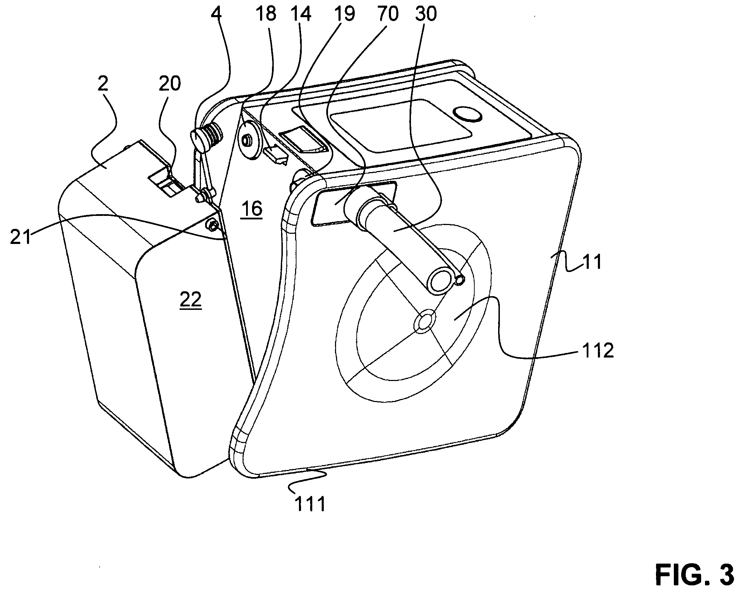

Das Pumpaggregatgehäuse 1 ist im wesentlichen quaderförmig ausgebildet, wobei es eine Rückwand 10, eine annähernd parallel dazu verlaufende vordere Wand 11, eine zwischen diesen Wänden angeordnete erste Seitenwand 16, eine annähernd parallel zur ersten Seitenwand 16 verlaufende zweite Seitenwand 17 sowie eine obere Wand 12 und eine in den Figuren nicht sichtbare untere Wand aufweist. Das Gehäuse 1 ist vorzugsweise aus Kunststoff oder Metall gefertigt. Die Rückwand 10 und die vordere Wand 11 können plan ausgebildet sein. Die vordere Wand 11 kann jedoch auch, wie in Figur 3 sichtbar ist, eine zentrale, nach aussen vorstehende Wölbung 112 aufweisen. Die Rückwand 10 kann nach innen gekrümmt ausgebildet sein, um sich so der Form des menschlichen Körpers anzupassen und damit besser am Körper anzuliegen. Auch die vordere Wand 11 kann entsprechend gekrümmt sein.

Vorzugsweise besitzen die Rückwand 10 und die vordere Wand 11 die grössten Wandflächen. Zudem sind die obere und untere Wand 12 länger ausgebildet als die Seitenwände 16, 17, so dass das Gehäuse 1 einen liegenden Quader bildet.

In den Figuren sind die Befestigungsmittel für entsprechende Klammern und Bänder zum Tragen der tragbaren bzw. portablen Absaugpumpeneinheit nicht dargestellt. Sie befinden sich jedoch vorzugsweise auf der Rückwand oder den Seitenwänden des Gehäuses.

Im Gehäuse 1 sind Bedienungselemente für das Pumpenaggregat 6 vorhanden. Vorzugsweise sind diese Elemente in der oberen Wand 12 angeordnet. Im hier dargestellten Beispiel ist ein Hauptschalter 5 vorhanden, um die Einheit bzw. das Gerät ein- und auszuschalten. Ferner ist ein Anzeige- und Bedienfeld 15 vorhanden, auf welchem Statusangaben zum Gerät, zum Saugvorgang und weitere für eine optimale Absaugung hilfreiche Angaben angezeigt bzw. abgefragt werden können. Beispielsweise kann die Luftdurchflussmenge durch die Sekretleitung 30 gemessen und im Anzeigefeld 15 dargestellt werden. Zudem ist es möglich, im Gehäuse 1 ein Datenspeicherelement anzuordnen, um Messdaten zu speichern und nach entsprechender Befehlseingabe im Anzeigefeld 15 wiederzugeben.

Zudem kann die Vakuumpumpe 62 bzw. der Motor 60 über dieses Feld 15 aktiviert bzw. gewünschte Absaugparameter eingegeben bzw. ausgewählt werden. Vorzugsweise ist das Feld 15 ein Touch-Screen Feld bekannter Art. Es ist jedoch auch möglich, anstelle eines derartigen Feldes Bedienungsknöpfe und Schalter und wahlweise einen bekannten LCD Display zu verwenden. Des weiteren können diese Elemente auch in einer anderen Wand angeordnet sein.

Die Rückwand 10 und die vordere Wand 11 stehen mit ihren Kanten mindestens der ersten Seitenwand 16, vorzugsweise beiden Seitenwänden und auch der unteren und oberen Wand vor. Das Feld 15 wird durch die vorstehenden Wände 10, 11 vor unerwünschter Betätigung geschützt.

Der Fluidsammelbehälter 2 ist ebenfalls annähernd quaderförmig ausgebildet. Er weist zwei annähernd zueinander parallel verlaufende und annähernd plane Wände 22 auf, welche die Vorder- und Rückwand bilden. Dasselbe gilt für die Seitenwände und die obere und untere Wand.

Dieser Fluidsammelbehälter 2 ist nun im Gehäuse 1 lösbar und vorzugsweise vollständig entfernbar gehalten. Hierfür weisen die Rückwand 10 und die vordere Wand 11 des Gehäuses Bereiche auf, hier vordere Wandkante 110 und hintere Wandkante 100 genannt, welche der ersten Seitenwand 16 vorstehen. Diese Wandkanten 100, 110 sind vorzugsweise geschwungen ausgebildet, wobei sie eine Einbuchtung zu ihrer eigenen Wandfläche hin aufweisen. Zwischen diesen Wandkanten 100, 110 ist der Behälter 2 gehalten, wobei die geschwungenen Bereiche das Ergreifen und manuelle Halten des Behälters 2 erleichtern.

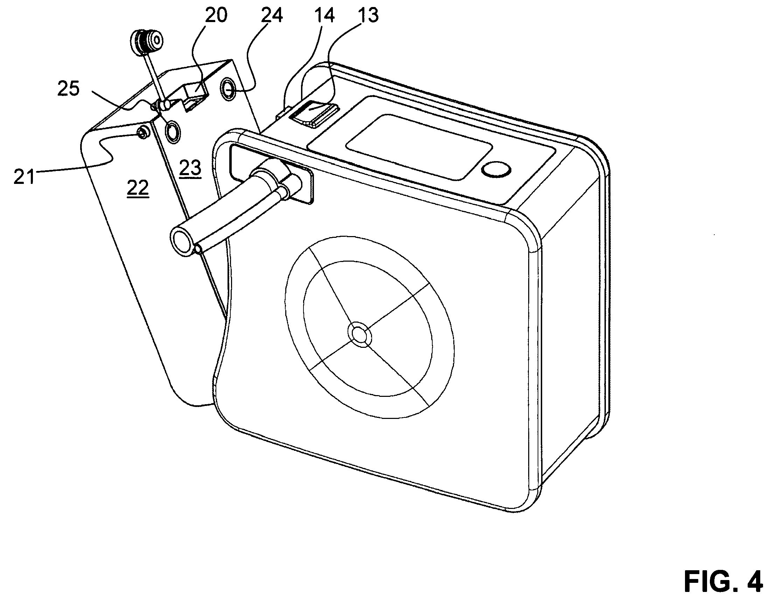

Wie in den Figuren 3 und 4 erkennbar ist, ist der Behälter ein- und ausschwenkbar zwischen den zwei Kanten 100, 110 angeordnet. Dabei ist er in seinem unteren Bereich zwischen den Wandkanten 100, 110 schwenkbar gehalten. Vorzugsweise lässt er sich in diese Lage einklinken. Hierfür können entsprechende Bolzen an der Vorder- und Rückwand 22 des Behälters 2 und entsprechende Ausnehmungen in den Wandkanten 100, 110 vorhanden sein. Es können natürlich auch die Bolzen in den Wandkanten 100, 110 und die Ausnehmungen im Behälter 2 angeordnet sein. Zudem sind auch andere Befestigungsarten, welche eine Schwenkbewegung und ein anschliessendes Entfernen des Behälters 2 erlauben, möglich. Mindestens in diesem Bereich ist die Form der Vorderund Rückwand 22 des Behälters 2 der Form der Wandkanten 100, 110 angepasst.

In einem oberen Bereich ist der Behälter 2 mit dem Gehäuse 1 verriegelbar. Hierfür weist der Behälter 2 eine Ausnehmung 20 auf, in welche eine Rückhaltenase 14 des Gehäuses 1 eingreifen kann. Die Rückhaltenase 14 lässt sich über einen Entriegelungsknopf oder -schalter 13 aus dem Eingriff mit der Ausnehmung 20 lösen, so dass der Behälter 2 ausgeschwenkt werden kann. Der Entriegelungsknopf 13 ist vorzugsweise in der oberen Wand 12 angeordnet. Als zusätzlicher Halt kann der Behälter 2 an seiner vorderen und seiner Rückwand mit vorstehenden Zapfen versehen sein, welche an die Wandkanten 100, 110 gepresst werden, so dass der Behälter 2 nach Lösen der Verriegelung noch nicht automatisch aus dem Gehäuse 1 fällt.

Zur Verbindung von Gehäuse 1 bzw. Aggregat 6 mit dem Fluidbehälter 2 sind im Gehäuse 1 ein gehäuseseitiger Vakuumanschluss 18 und der gehäuseseitige Sekretanschluss 19 angeordnet, wie dies in Figur 3 gut erkennbar ist. Die behälterseitigen Pendants sind in Figur 4 erkennbar. Die Bezugsziffer 24 bezeichnet den behälterseitigen Vakuumanschluss und die Bezugsziffer 25 den behälterseitigen Sekretanschluss. Beide Anschlüsse sind in einer Seitenwand 23 des Behälters 2 angeordnet. Die Vakuumanschlüsse 24, 18 bilden die Verbindung zwischen Vakuumpumpe 62 und Behälter 2. Die Sekretanschlüsse 19, 25 verbinden den Behälter 2 mit dem mit der Sekretleitung 30 verbindbaren Adapter 7.

Wird der Behälter 2 entfernt, so lässt sich der behälterseitige Anschluss 25 mittels eines Verschlusselements 4 verschliessen. Dieses ist vorzugsweise am Behälter 2 befestigt, wie dies in Figur 4 erkennbar ist. Es weist einen Steg und eine am Ende des Stegs angeordnete Verschlusskappe auf. Die Verschlusskappe ist geeignet, um den Anschluss 25 zu verschliessen. Der Anschluss 24 kann durch ein nicht dargestelltes Filter verschlossen werden, das beispielsweise bei Feuchtigkeitssättigung automatisch ganz schliesst. Andere Verschlussarten sind auch möglich.

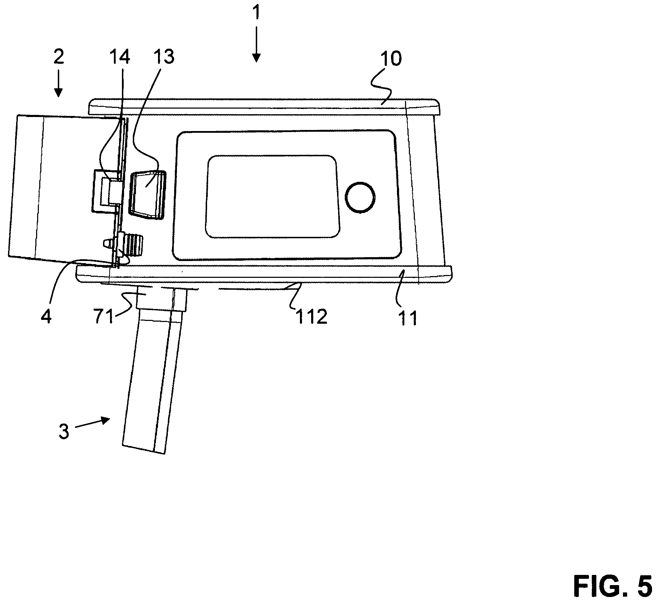

In Figur 2 ist erkennbar, dass vorstehende Kanten des Pumpaggregatgehäuses 1, nämlich eine hintere und eine vordere Bodenkante 101, 111, eine Standfläche zur Auflage auf einer Oberfläche, beispielsweise einem Tisch, bilden. Der Fluidsammelbehälter 2 endet jedoch vorzugsweise mit seinem unteren Boden oberhalb dieser Standfläche, so dass er frei im Gehäuse 1 hängt. Wie in Figur 5 erkennbar ist, ist er jedoch geführt zwischen den zwei Wandkanten 100, 110 gehalten. Zudem ist erkennbar, dass der Behälter 2 das Gehäuse 1 vorzugsweise nur auf einer einzigen Seite überragt.

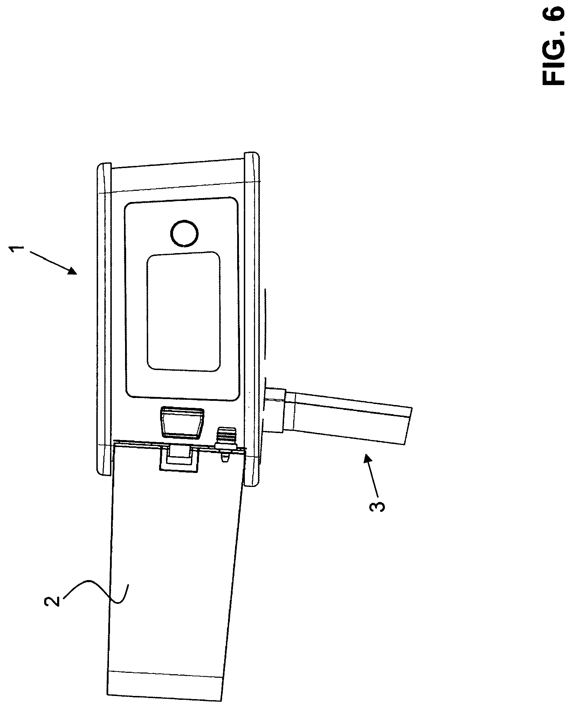

Die Grösse des Behälters 2 kann variiert werden. In Figur 5 ist ein relativ kurzer Behälter 2 dargestellt, in Figur 6 ein längerer Behälter 2. Sie müssen lediglich im Bereich zwischen den Wandkanten 100, 110 dieselbe Form aufweisen, damit beide in demselben Gehäuse 1 befestigt werden können. Die restliche Form kann frei gewählt werden.

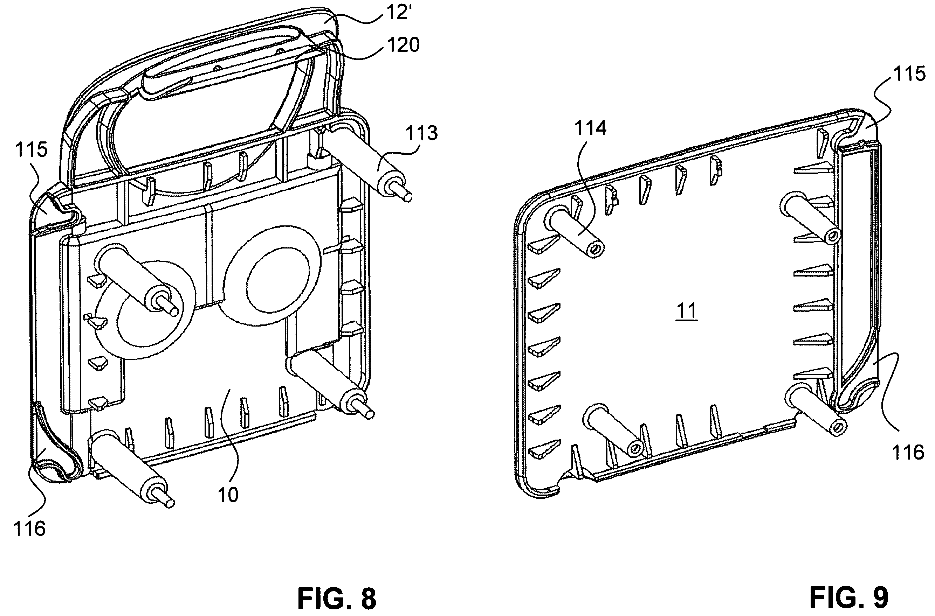

In den Figuren 8 und 9 sind zwei gegenüberliegende Teile eines Pumpaggregatgehäuses 1 gemäss einem dritten Ausführungsbeispiel dargestellt. Diese zwei Teile bilden die Rückwand 10 (Figur 8) und die vordere Wand 11 (Figur 9) des Gehäuses 1. Beide Teile sind vorzugsweise je einstückig im Spritzgussverfahren aus Kunststoff hergestellt.

Die zwei Teile 10, 11 sind ineinander steckbar gestaltet, wobei sie beabstandet zueinander gehalten sind. Hierzu sind an ihren Innenseiten senkrecht vorstehende Steckdorne 113 und dazu passende gegenüberliegende Aufnahmebuchsen 114 angeordnet. Auch diese sind vorzugsweise einstückig mit den Wänden gespritzt.

Eine oder beide der zwei Wände, hier die Rückwand 10, kann mit einem Handgriff 12' versehen sein. Am Handgriff 12' ist vorzugsweise eine Wanne 120 angeordnet, deren Wölbung nach oben offen ist. Diese Wanne 120 dient zur Aufnahme bzw. Befestigung des Patientenschlauchs 30, so dass dieser entlang der Pumpe geführt gehalten ist.

Mindestens ein Teil 10, 11, vorzugsweise beide Teile sind an ihrer stirnseitigen Kante mit einer oberen und einer unteren Kulissenführung 115, 116 versehen. Die zwei gegenüberliegenden oberen Kulissenführungen 115 weisen ein verbreiterte Einführungsöffnung und einen anschliessend horizontal verlaufenden Endbereich auf, welcher von der Kante weg nach innen gerichtet ist. Die zwei gegenüberliegenden unteren Kulissenführungen 116 weisen ebenfalls einen verbreiterten Eingangsbereich auf. Dieser geht jedoch ebenfalls von der Kante weg nach innen in einen tiefer gelegenen, schräg nach unten gerichteten Endbereich über. Diese Kulissenführungen 115 und 116 dienen der Aufnahme und Halterung des Fluidsammelbehälters 2.

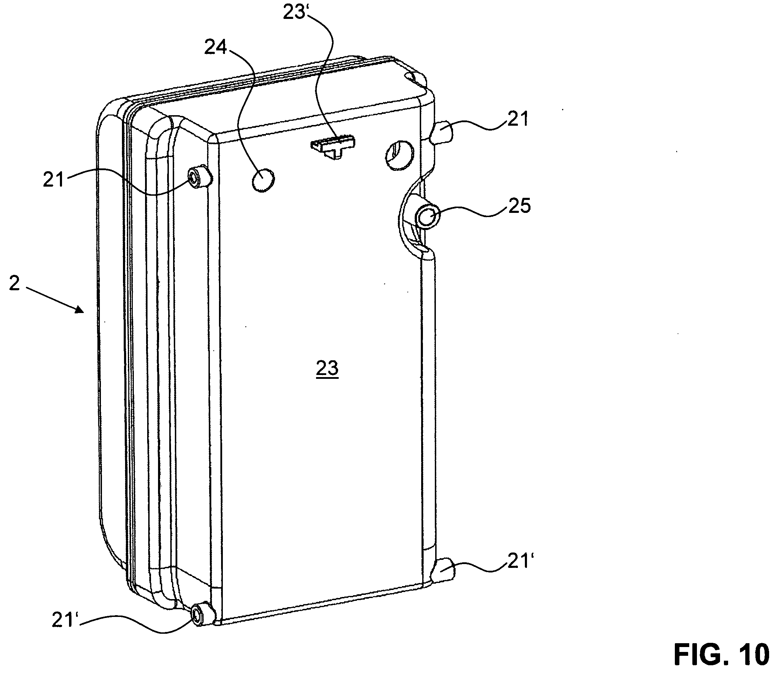

Ein entsprechender Fluidsammelbehälter 2 aus Kunststoff ist in Figur 10 dargestellt. Im Bereich einer Seitenwand 23 weist er obere und untere Bolzen oder Zapfen 21, 21' auf, welche einstückig an die Rückwand bzw. vordere Wand angespritzt sind und im wesentlichen senkrecht davon abstehen.

Um den Sammelbehälter 2 nun am Gehäuse 1 lösbar zu befestigen, wird er zuerst mit seinen unteren Bolzen 21' in die unteren Kulissenführungen 116 bis zum Anschlag eingeführt und anschliessend durch eine Schwenkbewegung um die durch die Endposition der unteren Kulissenführung 116 definierte Schwenkachse mit den oberen Bolzen 21 in die obere Kulissenführungen 115 bzw. in deren Endposition eingeklinkt. Dieselbe Art der Befestigung ist auch für die weiter oben erwähnten Ausführungsbeispiele bevorzugt. Die Zapfen können auch am Gehäuse und die Kulissenführungen am Behälter angeordnet sein. Andere Befestigungsarten sind jedoch möglich.

Wie in Figur 10 erkennbar ist, sind bei diesem Sammelbehälter 2 der behälterseitige Vakuumanschluss 24 und der behälterseitige Sekretanschluss 25 nicht mehr gleich ausgebildet wie im obigen Ausführungsbeispiel. Auch das Adapterstück 70 muss nicht zwingend in einer Vorder- oder Rückwand 10, 11 sondern kann auch an einer anderen Stelle des Gehäuses 1 angeordnet sein, beispielsweise in einer Stirnseite. Des weiteren ist der Behälter 2 anstatt mit einer Ausnehmung mit einer Eingriffsrippe 23' zur Fixierung des Behälters 2 am Gehäuse 1 versehen, in welchem die Rückhaltenase des Gehäuses 1 eingreifen kann. Nase und Ausnehmung bzw. Rippe lassen sich auch alternativ am Behälter bzw. am Gehäuse anordnen. Diese Merkmale lassen sich beliebig miteinander kombinieren und auch in den oben erwähnten Beispielen verwenden.

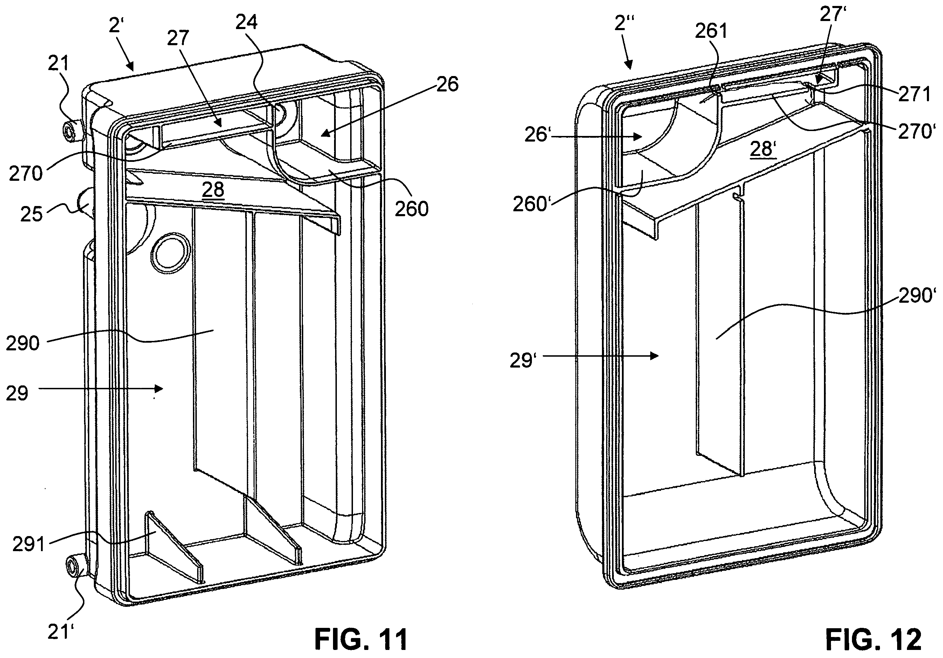

Der Behälter 2 kann mit einer einzigen Kammer versehen sein. Vorzugsweise ist sein Innenraum jedoch unterteilt gestaltet, wie dies in den Figuren 11 und 12 dargestellt ist. Dieser Behälter lässt sich für alle Ausführungsformen einsetzen. Zudem eignet er sich auch für andere Arten von Drainagepumpen.

Der Behälter 2 besteht aus zwei sich zu einem gemeinsamen Behälter ergänzenden Spritzgussteilen 2', 2" aus Kunststoff. Sie sind vorzugsweise transparent gestaltet. Die zwei Teile 2', 2" können zusammengesteckt und gegebenenfalls miteinander verschweisst werden. Beide Teile 2', 2" sind im Innenraum mit diversen Rippen versehen, welche im folgenden detailliert beschrieben werden. Die zwei Teile 2', 2" weisen deckungsgleiche Rippen auf, so dass sie beim Zusammenfügen gemeinsame Kammern und Bereiche bilden. Die Rippen werden beim Zusammenfügen vorzugsweise miteinander verschweisst oder verklebt, so dass eine luft- und flüssigkeitsdichte Verbindung entsteht.

Der behälterseitige Vakuumanschluss 24 ist im oberen Bereich des Behälters 2 angeordnet, vorzugsweise in der Seitenwand 23, welche seitlich mit den Zapfen 21,21' zum Einklinken in das Gehäuse 1 versehen ist. Der Vakuumanschluss 24 ist durch eine durchgehende Öffnung in dieser Seitenwand 23 gebildet. Die Öffnung 24 führt in eine Vakuumkammer 26, 26', welche vom restlichen Innenraum des Behälters 2 bis auf einen Vakuumdurchgang 261 vollständig dicht getrennt ist. Dies wird durch eine erste gebogene Rippe 260 im ersten Teil 2' und eine dazu gleich geformte zweite Rippe 260' im zweiten Teil 2" erwirkt. Der Durchgang 261 kann im ersten oder wie hier dargestellt im zweiten Teil 2" angeordnet sein oder er kann an der Verbindungsstelle der zwei Rippen 260, 260' angeordnet sein. Der Vakuumdurchgang 261 ist vorzugsweise im oberen Bereich, benachbart zur oberen Wandung des Behälters 2, angeordnet.

Anschliessend an diese Vakuumkammer 26, 26' folgt, ebenfalls entlang der oberen Wandung verlaufend, eine Zwischenkammer 27, 27'. Der Vakuumdurchgang 261 verbindet die Vakuumkammer 26, 26' mit der Zwischenkammer 27, 27'. Die Zwischenkammer 27, 27' ist vorzugsweise durch eine rechtwinklig gebogene dritte Rippe 270 im ersten Teil 2' und eine deckungsgleiche Rippe 270' im zweiten Teil 2" gebildet. In einem der zwei Teile bzw. im Zwischenbereich ist wiederum ein Durchgang, hier Zwischendurchgang 271 genannt, vorhanden, welche die Zwischenkammer 27, 27' mit dem restlichen Innenraum des Behälters 2 verbindet. Der Zwischendurchgang 271 befindet sich vorzugsweise in einem von der Vakuumkammer 26, 26' fernen Bereich.

Die zwei Durchgänge 261 und 271 sind relativ schmal ausgebildet. Es können aber auch mehrere Durchgänge vorhanden sein. Sie sollten klein genug sein, um ein Rückfliessen des Sekrets bzw. des abgesaugten Fluids möglichst zu verhindern und gross genug, damit der Behälter möglichst schnell genug mit dem angelegten Unterdruck beaufschlagt werden kann.

Unterhalb der Zwischenkammer 27, 27' und unterhalb des Zwischendurchgangs 271 ist in beiden Teilen 2', 2" je eine Schrägrippe 28, 28' angeordnet, welche sich vom Zwischendurchgang 271 zur Vakuumkammer 26, 26' hin nach unten erstreckt. Die Schrägrippen 28, 28' unterteilen der Innenraum in einen oberen und unteren Bereich, wobei der obere Bereich ein wesentlich kleineres Volumen umfasst als der untere Bereich. Die Schrägrippen (28, 28') erstrecken sich vorzugsweise gemeinsam über einen wesentlichen Teil der Breite, aber nicht über die gesamte Breite des Behälters (2) und über die gesamte Tiefe. Dadurch muss das angesaugte Fluid der Schrägrippe 28, 28' entlang nach unten fliessen.

Der untere Bereich kann noch mit vertikal verlaufenden Trennrippen 290, 290', 291 versehen sein, welche sich über fast die gesamte Höhe des unteren Bereichs oder nur über einen kurzen unteren Teil davon erstrecken können.

Der Sekretanschluss 25 ist im unteren Bereich angeordnet. Der unterer Bereich dient somit zur Aufnahme des abgesaugten Fluids und bildet eine Sekretkammer 29, 29'. Die Trennrippen 290, 290', 291 unterteilen diese Kammer in Teilkammern, welche miteinander fluidmässig verbunden sind. Sie vermeiden dabei jedoch ein Hin- und Herschwappen des gesammelten Fluids. Die Schrägrippen 28, 28' verhindern, dass das Fluid in den oberen Bereich spritzt bzw. dass bei leichten Schräglagen des Behälters das Fluid in diesen Bereich fliessen kann. Die schmalen Durchgangsöffnungen und insbesondere die labyrinthartige Anordnung dank der Zwischen- bzw. Expansionskammer verhindern, dass Fluid, welches trotzdem in den oberen Bereich gelangt ist, bis zum Vakuumanschluss vordringen kann.

In einer nicht dargestellten Ausführungsform sind die Rippen lediglich in einem Teil angeordnet und der zweite Teil ist flach ausgebildet und dient als Deckel.

Die Zwischenkammer 27, 27' ist optional aber bevorzugt, da dadurch eine direkte Verbindung zwischen Vakuumkammer 26, 26' und Sekretkammer 29, 29' vermieden wird.

Alle oben beschriebenen Fluidsammelbehälter lassen sich in unterschiedlichen Grössen herstellen.

### Die Erfindung betrifft ferner:

Eine tragbare Absaugpumpeneinheit zum Absaugen von Körperflüssigkeiten und/oder Luft, wobei die Absaugpumpeneinheit ein Pumpaggregatgehäuse (1) mit einem Pumpaggregat (6) und mindestens einen lösbar mit diesem Pumpaggregatgehäuse (1) verbundenen Fluidsammelbehälter (2) aufweist, wobei das Pumpaggregatgehäuse (1) eine vordere Wand (11), eine Rückwand (10) und eine zwischen diesen zwei Wänden (10, 11) angeordnete Seitenwand (16) aufweist, dadurch gekennzeichnet, dass die vordere Wand (11) und die Rückwand (10) je eine Wandkante (110, 100) aufweisen, welche dieser Seitenwand (16) vorsteht und dass der Fluidsammelbehälter (2) zwischen diesen Wandkanten (110, 100) gehalten ist.

Diese Absaugpumpeneinheit, wobei der Fluidsammelbehälter (2) zwischen den Wandkanten (110, 100) ein- und ausschwenkbar ist.

Diese Absaugpumpeneinheit, wobei der Fluidsammelbehälter (2) in einem unteren Bereich zwischen den Wandkanten (110, 100) schwenkbar gehalten und in einem oberen Bereich mit dem Pumpaggregatgehäuse (1) verriegelbar ist.

Diese Absaugpumpeneinheit, wobei eine Rückhaltenase (14) vorhanden ist, welche über ein Entriegelungselement (13) aus einem den Fluidsammelbehälter (2) fixierenden Eingriff lösbar ist.

Diese Absaugpumpeneinheit, wobei der Fluidsammelbehälter (2) im unteren Bereich in das Pumpaggregatgehäuse (1) einklinkbar ist.

Diese Absaugpumpeneinheit, wobei der Behälter (2) vorstehende Zapfen (21, 21') und das Gehäuse (1) seitliche Kulissenführungen (115, 116) aufweisen, welche im Eingriff miteinander bringbar sind.

Diese Absaugpumpeneinheit, wobei das Pumpaggregatgehäuse (1) eine Standfläche zur Auflage auf einer Oberfläche aufweist und wobei der Fluidsammelbehälter (2) mit seinem unteren Boden oberhalb dieser Standfläche endet.

Diese Absaugpumpeneinheit, wobei das Pumpaggregatgehäuse (1) im wesentlichen quaderförmig ausgebildet ist.

Diese Absaugpumpeneinheit, wobei das Pumpaggregat (6) mindestens einen Motor (60) und eine Vakuumpumpe (62) umfasst, wobei die Vakuumpumpe (62) eine Membranpumpe ist und wobei das Pumpaggregat (6) annähernd zentral im Pumpaggregatgehäuse (1) angeordnet ist.

Diese Absaugpumpeneinheit, wobei der Fluidsammelbehälter (2) das Pumpaggregatgehäuse (1) nur auf einer einzigen Seite überragt.

Diese Absaugpumpeneinheit, wobei ein Anschluss für einen Patientenschlauch (3) in der vorderen Wand (11) angeordnet ist.

Diese Absaugpumpeneinheit, wobei in der Seitenwand (16) des Pumpaggregatgehäuses (1) ein Vakuumanschluss (18) und ein Sekretanschluss (19) angeordnet sind.

Diese Absaugpumpeneinheit, wobei ein Adapter (7) für einen Patientenschlauch (3) vorhanden ist, wobei der Adapter (7) in eine Wand (11) des Pumpaggregatgehäuses (1) einführbar ist und wobei der Adapter (7) mit dem Pumpaggregat (6) und dem Fluidsammelbehälter (2) verbindbar ist.

Diese Absaugpumpeneinheit, wobei der Anschluss bzw. der Adapter (7) zur Aufnahme eines doppellumigen Patientenschlauchs (3) bestehend aus Sekretschlauch (30) und Messschlauch (31) ausgebildet ist.

Einen Fluidsammelbehälter zur Aufnahme eines abgesaugten Fluids, insbesondere zur Verwendung mit einer Absaugpumpeinheit wie oben dargelegt, wobei der Sammelbehälter einen Sekretanschluss (24) zum Anschluss eines patientenseitigen Drainageschlauchs und einen Vakuumanschluss (25) zum Anschluss an eine Saugpumpe aufweist und wobei der Behälter einen Innenraum aufweist, welcher mittels Rippen unterteilt ist, dadurch gekennzeichnet, dass der Innenraum mindestens in eine Vakuumkammer (26, 26') und eine Sekretkammer (29, 29') unterteilt ist, wobei diese zwei Kammern über mindestens einen Durchgang miteinander verbunden sind und wobei der Vakuumanschluss (24) in der Vakuumkammer (26, 26') und der Sekretanschluss (25) in der Sekretkammer (29, 29') angeordnet sind.

Diesen Fluidsammelbehälter, wobei zwischen Vakuumkammer (26, 26') und Sekretkammer (29, 29') eine Zwischenkammer (27, 27') angeordnet ist und wobei von der Vakuumkammer (26, 26') ein Vakuumdurchgang (261) zur Zwischenkammer (27, 27') und von dort ein Zwischendurchgang (271) zur Sekretkammer (29, 29') verläuft.

Diesen Fluidsammelbehälter, wobei keine direkte Verbindung zwischen Vakuumkammer (26, 26') und Sekretkammer (29, 29') existiert.

Diesen Fluidsammelbehälter, wobei die Vakuumkammer (26, 26') im oberen Bereich des Behälters (2) angeordnet ist.

Diesen Fluidsammelbehälter, wobei im oberen Bereich der Sekretkammer (29, 29'), benachbart zur Vakuumkammer (26, 26'), eine Schrägrippe (28, 28') angeordnet ist, welche sich über einen wesentlichen Teil der, aber nicht über die gesamte Breite des Behälters (2) erstreckt und welche sich aber über die gesamte Tiefe erstreckt.

Diesen Fluidsammelbehälter, wobei die Sekretkammer (29, 29') durch Vertikalrippen (290, 290', 291) in miteinander verbundene Teilkammern unterteilt ist.

Die erfindungsgemässe Absaugpumpeneinheit erlaubt ein einfaches und sicheres Auswechseln des Fluidsammelbehälters und ermöglicht dem Patienten eine erhöhte Mobilität.

### Bezugszeichenliste

- 1: Pumpaggregatgehäuse
- 10: Rückwand
- 100: Hintere Wandkante
- 101: Hintere Bodenkante
- 11: Vordere Wand
- 110: Vordere Wandkante
- 111: Vordere Bodenkante
- 112: Wölbung
- 113: Steckdorn
- 114: Aufnahmebuchse
- 115: obere Kulissenführung
- 116: untere Kulissenführung
- 12: obere Wand
- 12': Handgriff
- 120: Wanne
- 13: Entriegelungsknopf
- 14: Rückhaltenase
- 15: Anzeige- und Bedienfeld
- 16: erste Seitenwand
- 17: zweite Seitenwand
- 18: gehäuseseitiger Vakuumanschluss
- 19: gehäuseseitiger Sekretanschluss

- 2: Fluidsammelbehälter
- 2': erster Teil
- 2": zweiter Teil
- 20: Ausnehmung
- 21: oberer Zapfen
- 21': unterer Zapfen
- 22: Rückwand
- 23: Seitenwand
- 23': Eingriffrippe
- 24: behälterseitiger Vakuumanschluss
- 25: behälterseitiger Sekretanschluss
- 26: erster Teil der Vakuumkammer
- 26': zweiter Teil der Vakuumkammer
- 260: erste Rippe
- 260': zweite Rippe
- 261: Vakuumdurchgang
- 27: erster Teil der Zwischenkammer
- 27': zweiter Teil der Zwischenkammer
- 270: dritte Rippe
- 270': vierte Rippe
- 271: Zwischendurchgang
- 28: erste Schrägrippe
- 28': zweite Schrägrippe
- 29: erster Teil der Sekretkammer
- 29': zweiter Teil der Sekretkammer
- 290: erste lange Trennrippe
- 290': zweite lange Trennrippe
- 291: kurze Trennrippe

- 3: Patientenschlauch
- 30: Sekretleitung
- 31: Messleitung

- 4: Verschlusselement

- 5: Hauptschalter

- 6: Pumpaggregat
- 60: Motor
- 61: Batterie
- 62: Vakuumpumpe

- 7: Adapter
- 70: Flansch
- 71: Rohranschlussstück
- 72: Kopplungsteil
- 73: Verbindungsteil
- 74: Ende

## Patentansprüche

1. Tragbare Absaugpumpeneinheit zum Absaugen von Körperflüssigkeiten und/oder Luft, wobei die Absaugpumpeneinheit ein Pumpaggregatgehäuse (1) mit einem Pumpaggregat (6) und mindestens einen lösbar mit diesem Pumpaggregatgehäuse (1) verbundenen Fluidsammelbehälter (2) aufweist, wobei das Pumpaggregatgehäuse (1) eine vordere Wand (11), eine Rückwand (10) und eine zwischen diesen zwei Wänden (10, 11) angeordnete Seitenwand (16) aufweist, wobei ein Adapter (7) für einen Patientenschlauch (3) vorhanden ist, **dadurch gekennzeichnet, dass** der Adapter (7) in eine Wand (10, 11, 16) des Pumpaggregatgehäuses (1) einführbar ist und dass der Adapter (7) mit dem Fluidsammelbehälter (2) verbindbar ist.

2. Absaugpumpeneinheit nach Anspruch 1, wobei der Adapter (7) zur Aufnahme eines doppellumigen Patientenschlauchs (3) bestehend aus Sekretschlauch (30) und Messschlauch (31) ausgebildet ist.

3. Absaugpumpeneinheit nach einem der Ansprüche 1 bis 2, wobei in der Seitenwand (16) des Pumpaggregatgehäuses (1) ein Vakuumanschluss (18) und ein Sekretanschluss (19) angeordnet sind.

4. Absaugpumpeneinheit nach Anspruch 1, wobei der Adapter (7) in der vorderen Wand (11) angeordnet ist.

5. Absaugpumpeneinheit nach einem der Ansprüche 1 bis 4, wobei die vordere Wand (11) und die Rückwand (10) je eine Wandkante (110, 100) aufweisen, welche dieser Seitenwand (16) vorsteht und dass der Fluidsammelbehälter (2) zwischen diesen Wandkanten (110, 100) gehalten, insbesondere zwischen den Wandkanten (110, 100) ein- und ausschwenkbar, ist.

6. Absaugpumpeneinheit nach Anspruch 5, wobei der Fluidsammelbehälter (2) in einem unteren Bereich zwischen den Wandkanten (110, 100) schwenkbar gehalten und in einem oberen Bereich mit dem Pumpaggregatgehäuse (1) verriegelbar ist.

7. Absaugpumpeneinheit nach einem der Ansprüche 1 bis 6, wobei das Pumpaggregatgehäuse (1) im wesentlichen quaderförmig ausgebildet ist.

8. Absaugpumpeneinheit nach einem der Ansprüche 1 bis 7, wobei das Pumpaggregat (6) mindestens einen Motor (60) und eine Vakuumpumpe (62) umfasst, wobei die Vakuumpumpe (62) eine Membranpumpe ist und wobei das Pumpaggregat (6) annähernd zentral im Pumpaggregatgehäuse (1) angeordnet ist.

9. Absaugpumpeneinheit nach einem der Ansprüche 1 bis 8, wobei der Fluidsammelbehälter (2) das Pumpaggregatgehäuse (1) nur auf einer einzigen Seite überragt.

10. Absaugpumpeneinheit nach einem der Ansprüche 1 bis 9, wobei der Adapter (7) einen Flansch (70) aufweist zur Befestigung des Adapters (7) im Pumpengehäuse (1).

11. Absaugpumpeneinheit nach Anspruch 1, wobei der Adapter (7) mit dem Pumpaggregat (6) verbindbar ist.

## Claims

1. A portable aspiration pump unit for the aspiration of bodily fluids and/or air, wherein the aspiration pump unit has a pump assembly housing (1) with a pump assembly (6) and at least one fluid collection reservoir (2) detachably connected to this pump assembly housing (1), wherein the pump assembly housing (1) has a front wall (11), a rear wall (10), and a side wall (16) arranged between these two walls (10, 11), wherein an adapter (7) for a patient's tube (3) is present, **characterized in that** the adapter (7) can be introduced into a wall (10, 11, 16) of the pump assembly housing (1) and that the adapter (7) can be connected to the fluid collection reservoir (2).

2. The aspiration pump unit as claimed in claim 1, wherein the adapter (7) is configured to accommodate a double-lumen patient's tube (3) consisting of secretion tube (30) and metering tube (31).

3. The aspiration pump unit as claimed in one of claims 1 to 2, wherein a vacuum connector (18) and a secretion connector (19) are arranged in the side wall (16) of the pump assembly housing (1).

4. The aspiration pump unit as claimed in claim 1, wherein the adapter (7) is arranged in the front wall (11).

5. The aspiration pump unit as claimed in one of claims 1 to 4, wherein the front wall (11) and the rear wall (10) each have one wall edge (110, 100) projecting beyond this side wall (16) and the fluid collection reservoir (2) is held between these wall edges (110, 100), particularly wherein the fluid collection reservoir (2) can swivel in and out between the wall edges (110, 100).

6. The aspiration pump unit as claimed in claim 5, wherein the fluid collection reservoir (2) is held and hinged in a lower region between the wall edges (110, 100) and can be locked to the pump assembly housing (1) in an upper region.

7. The aspiration pump unit as claimed in one of claims 1 to 6, wherein the pump assembly housing (1) is formed essentially as a cuboid.

8. The aspiration pump unit as claimed in one of claims 1 to 7, wherein the pump assembly (6) comprises at least a motor (60) and a vacuum pump (62), wherein the vacuum pump (62) is a membrane pump and wherein the pump assembly (6) is arranged approximately central in the pump assembly housing (1).

9. The aspiration pump unit as claimed in one of claims 1 to 8, wherein the fluid collection reservoir (2) projects beyond the pump assembly housing (1) on only a single side.

10. The aspiration pump unit as claimed in one of claims 1 to 9, wherein the adapter (7) comprises a flange (70) for securing the adapter (7) in the housing (1).

11. The aspiration pump unit as claimed in claim 1, wherein the adapter (7) can be connected to the pump assembly (6).

## Revendications

1. Unité de pompe d'aspiration portable pour aspirer des liquides corporels et/ou d'air, l'unité de pompe d'aspiration présentant un boîtier d'ensemble de pompe (1) ayant une unité de pompage (6) et au moins un récipient collecteur de fluides (2) connecté de façon amovible à ce boîtier d'ensemble de pompe (1), le boîtier d'ensemble de pompe présentant une paroi avant (11), une paroi arrière (10) et une paroi latérale (16) disposée entre ces deux parois (10, 11), un adaptateur (7) pour un tube de patient (3) étant présent, **caractérisée en ce que** l'adaptateur (7) est insérable dans une paroi (10, 11, 16) du boîtier d'ensemble de pompe (1) et que l'adaptateur est connectable avec le récipient collecteur de fluides (2).

2. Unité de pompe d'aspiration selon la revendication 1, dans laquelle l'adaptateur (7) est formé pour recevoir un tube de patient (3) à double lumière consistant d'un tube à sécrétions (30) et un tube de mesure (31).

3. Unité de pompe d'aspiration selon une des revendications 1 à 2, dans laquelle un connecteur à vide (18) et un connecteur à sécrétion (19) sont arrangés dans la paroi latérale (16) du boîtier d'ensemble de pompe (1).

4. Unité de pompe d'aspiration selon la revendication 1, dans laquelle l'adaptateur (7) est arrangé dans la paroi avant (11).

5. Unité de pompe d'aspiration selon une des revendications 1 à 4, dans laquelle la paroi avant (11) et la paroi arrière (10) comprennent chacune un bord de paroi (110, 100), qui dépasse cette paroi latérale (16) et que le récipient collecteur de fluides (2) est maintenu entre ces deux bords de paroi (110, 100), en particulier entre les bords de paroi (110, 100) de manière à rentrer et sortir par pivotement.

6. Unité de pompe d'aspiration selon la revendication 5, dans laquelle le récipient collecteur de fluides (2) est maintenu entre les bords de paroi (110, 100) dans un domaine inférieur de manière pivotable et dans un domaine supérieur verrouillable avec le boîtier d'ensemble de pompe (1).

7. Unité de pompe d'aspiration selon une des revendications 1 à 6, dans laquelle le boîtier d'ensemble de pompe (1) est formé essentiellement en forme de cube.

8. Unité de pompe d'aspiration selon une des revendications 1 à 7, dans laquelle le groupe de pompage (6) comprend au moins un moteur (60) et une pompe à vide (62), dans laquelle la pompe à vide (62) est une pompe à membrane et dans laquelle le groupe de pompage (6) est arrangé approximativement de manière centrale dans le boîtier d'ensemble de pompe (1).

9. Unité de pompe d'aspiration selon une des revendications 1 à 8, dans laquelle le récipient collecteur de fluides (2) dépasse le boîtier d'ensemble de pompe (1) sur un seul côté.

10. Unité de pompe d'aspiration selon une des revendications 1 à 9, dans laquelle l'adaptateur (6) présente une bride (70) pour fixer l'adaptateur (7) dans le boîtier de pompe (1).

11. Unité de pompe d'aspiration selon la revendication 1, dans laquelle l'adaptateur (7) est connectable avec le groupe de pompage (6).
